(19) Europäisches Patentamt / European Patent Office / Office européen des brevets

(11) **EP 3 731 239 A1**

(12) **EUROPEAN PATENT APPLICATION**

(43) Date of publication:
28.10.2020 Bulletin 2020/44

(51) Int Cl.:
**G16H 70/40** *(2018.01)*

(21) Application number: 20164511.6

(22) Date of filing: 20.03.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 24.04.2019 US 201962838074 P
25.06.2019 US 201916451709

(71) Applicant: **Accenture Global Solutions Limited**
**Dublin 4 (IE)**

(72) Inventors:
• **UL AIN, Qurrat**
  **Dublin, D02 EH92 (IE)**
• **COSTABELLO, Luca**
  **Newbridge, DK W12 TR22 (IE)**

(74) Representative: **Boult Wade Tennant LLP**
**Salisbury Square House**
**8 Salisbury Square**
**London EC4Y 8AP (GB)**

(54) **POLYPHARMACY SIDE EFFECT PREDICTION WITH RELATIONAL REPRESENTATION LEARNING**

(57)     A system adapted to receive a knowledge base, which may include drug data, human biological data, drug-drug interactions, protein-protein interactions, gene expression, protein and drug interaction data, genotypic information for cell lines, drug side effects, and disease classification labels. The system may generate a knowledge graph based on the knowledge base, and convert the knowledge graph into embeddings that include points in a k-dimensional metric space. The system may determine a medical effect weighting based on a drug combination query, and update the embeddings of the drug combination. The system may utilize a pooling method to update predicate embeddings. The system may determine polypharmacy scores for the embeddings, and rank the predicted links between a drug combination and side effects.

Figure 1

## Description

### CROSS-REFERENCE TO RELATED APPLICATION(S)

This application claims benefit to U.S. Provisional Patent Application No. 62/838,074, filed on April 24, 2019, the entirety of which is incorporated by reference herein.

### FIELD OF THE INVENTION

The present disclosure relates in general to the fields of bioinformatics and embedding space generation, and in particular to methods and systems for predicting drug side effects of drug combinations using embedding space generated from a knowledge graph by modeling medical effect weighting and scoring predictions based on drug molecular-structure data and drug side-effect data.

### BACKGROUND

Basic techniques and equipment for machine learning, modeling data, graph embedding, and ranking drug compounds based on experimental data are known in the art. Enterprise systems have access to large volumes of information, both proprietary and public, relating to gene expression, drug interactions, molecular structures, and disease classification. Existing analytical applications and data warehousing systems have not been able to fully utilize such information. Often times, drug-related information is simply aggregated into large data warehouses without the inclusion of an added layer of relationship data connecting the information. Such aggregation of large amounts of data, without contextual or relational information, are data dumps that are not useful. Drug-related information stored in data warehouses are likely to exist in their original format, thus expending large amounts of computing resources to transform the information into searchable data in order to respond to a query.

Traditional approaches for searching drug-related data typically entail using string matching mechanisms. However, such previous approaches are limited in their ability to provide queried data. Moreover, most of the stored data is not easily searchable or available for analytics. Accordingly, conventional knowledge query systems return results that do not provide a useful picture of available data, requiring extra consumption of computing resources as knowledge queries are repeated and return inaccurate or incomplete results.

Drug-related data may be stored in different data stores depending on factors including data structure, volatility, volume, or other measurable attribute. These data stores may be designed, managed, and operated by different units within an enterprise organization. In practice, such data stores behave as data silos that are disparate, isolated, and make data less accessible across the units. More transparent and open data storage solutions are desired by pharmacological organizations to more efficiently and effectively share, access and analyze information. A multi-relational link prediction is desired to more efficiently and effectively identify side effects for drug combinations using machine-learning methods.

### BRIEF SUMMARY

The present disclosure may be embodied in various forms, including without limitation a system, method or computer-readable medium to predict the relationship or association between a drug side effect and a drug combination. In some embodiments, pre-existing drug data and human biological data may be used to generate a knowledge graph. Some embodiments of the present disclosure may include graphical representations that employ a knowledge base comprising drug data, human biological data, drug-drug interactions, protein-protein interactions, gene expression, protein and drug interaction data, genotypic information for cell lines, drug side effects, and disease classification labels. A schema for a knowledge graph may be utilized to graphically represent statements, expressed in a triples format, describing the knowledge base in order to extract sought-after information concerning the relationships between the information resourced in the knowledge base.

The graphical representation may be utilized to generate an embedding space. The embedding space may include nodes, which may represent the sets of triples that describe the relationship or predicate between a subject and an object. Accordingly, the nodes may represent embedding vectors corresponding to the structured datasets that describe the knowledge base. The inclusion of additional layers of relationship data may enable the modeling of medical effect weighting for a drug combination in order to score the prediction of the relationship between a drug combination and a drug side effect. Medical effect weighting may be utilized in a neural network, and may be selected from a combination-index database based on a drug combination query, in accordance with certain embodiments. In embodiment, the weighting of medical effect may utilize the Loewe additivity model and/or the Bliss independence model for evaluating, indexing and scoring drug combinations and their interactions.

A polypharmacy scoring function may rank the predicted links between the drug combinations and their potential side effects. The structural information of drug compounds, their associated biological data, and their effectiveness may be used to model drug combinations and determine side effect predictions. In additional to ranking the link prediction that a drug combination has a side effect, the benefits of this disclosure may include a reduction in the time spent during experimental testing by identifying side effects for certain drug combinations. Embodiments may enable a user to input a drug combination query and receive a polypharmacy score based on the medical effect weighting for the drug combination.

## BRIEF DESCRIPTION OF THE DRAWINGS

The foregoing and other objects, features, and advantages for embodiments of the present disclosure will be apparent from the following more particular description of the embodiments as illustrated in the accompanying drawings, in which reference characters refer to the same parts throughout the various views. The drawings are not necessarily to scale, emphasis instead being placed upon illustrating principles of the present disclosure.

Figure 1 is a schematic diagram illustrating a knowledge graph schema, in accordance with certain embodiments of the present disclosure.
Figure 2 is a block diagram illustrating an embodiment of a system for implementing polypharmacy scoring, in accordance with certain embodiments of the present disclosure.
Figure 3 is a block diagram illustrating an embodiment of a computer architecture for a computer device for implementing polypharmacy scoring, in accordance with certain embodiments of the present disclosure.
Figure 4 is a flow diagram illustrating the use of knowledge graph for polypharmacy scoring to identify a link prediction, in accordance with certain embodiments of the present disclosure.
Figure 5 is a flowchart illustrating an embodiment for determining polypharmacy scores for embeddings, in accordance with certain embodiments of the present disclosure.
Figure 6 is a flowchart illustrating an embodiment for generating candidate statements and probability metrics for the association of side effects with drug combinations to determine polypharmacy scores, in accordance with certain embodiments of the present disclosure.
Figure 7 is a flowchart illustrating an embodiment for generating polypharmacy scores based on a knowledge graph, a drug combination query, combination-index data, in accordance with certain embodiments of the present disclosure.

## DETAILED DESCRIPTION OF EMBODIMENTS OF THE INVENTION

Reference will now be made in detail to the embodiments of the present disclosure, examples of which are illustrated in the accompanying drawings.

The present disclosure may be embodied in various forms, including a product, a system, a method and a computer readable medium for polypharmacy scoring via knowledge graph embeddings based on drug molecular-structure data and drug side-effect data for a drug combination in order to predict side effect. A knowledge base **1** of drug-related data and associated relationships may be represented in a meaningful and understandable manner via knowledge graphs **2,** in accordance with certain embodiments. The model for a knowledge graph **2** may be defined by a schema or layout **3** that describes the data structures **4** and their relationships **5,** which may be represented by nodes **4'** and edges **5'** in the knowledge graph **2**. The knowledge graph **2** may present complex and innovative graphical structures that represent the relevant information in response to a query. In an embodiment, the knowledge graph **2** may represent the knowledge base **1** via graphical representations that correspond to structured data points or entities **4** (represented by nodes **4'**), relationships **5** (represented by edges **5'**), and attributes **6** (represented by node properties or edge properties **6'**) with semantic meaning. The graphically represented data offered by the knowledge graph **2** may provide semantic meaning for the knowledge base **1** by modeling the data via ontology, such as a schema **3.**

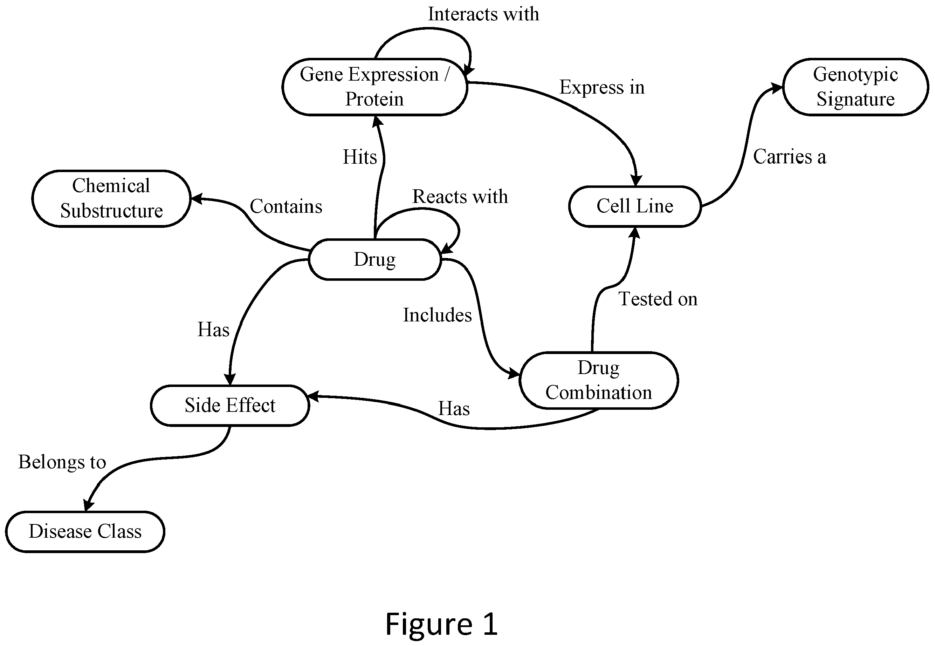

**Figure 1** illustrates a knowledge graph schema **3** within the scope of drug side effects and associated information, in accordance with certain embodiments of the present disclosure. The schema **3** may describe specific concepts or categories, *e.g.* classes in object-oriented data models or programming languages. The schema **3** may provide a manner to express statements **17** about the resources or knowledge base **1** using specific properties that describe the knowledge base **1**. For example, a Resource Description Framework (RDF) may provide a data model that represents the knowledge base **1** in expressions of the form subject-predicate-object, known as triples **7**. The subject may denote the resource, and the predicate may denote traits or aspects of the resource and express a relationship **5** between the subject and the object. For example, the statement "the drug combination has the side effect" **17** in the RDF model may be represented as the triple **7:** a subject denoting the "Drug Combination"; a predicate denoting "Has"; and an object denoting "Side Effect."

A collection of RDF statements **17** may be represented by a knowledge graph **2** having labeled vertices or nodes **4'**, and labeled edges that may have a distinguished direction. Such knowledge graphs **2** may be complex multigraphs that include multiple adjacencies or edges, including self-loops, between multiple vertices or nodes **4'**. For example, a side effect may be associated with a single drug or a drug combination. A data structure **4** may have properties **6,** which may be represented as subjects or objects of triples **7**, as defined by the knowledge graph schema **3**.

**Table 1** shows an example RDF-based layout or schema **3** that captures a triple **7** including a subject, predicate (*e.g.*, relationship **5**), and object, in accordance with certain embodiments of the present disclosure.

**TABLE 1**

| Subject | Predicate | Object |
|---|---|---|
| Drug | Contains | Chemical Structure |
| Drug | Hits | Gene / Protein |
| Drug | Reacts With | Drug |
| Drug | Has | Side Effect |
| Drug Combination | Includes | Drug |
| Drug Combination | Has | Side Effect |
| Side Effect | Belongs To | Disease Class |
| Drug Combination | Test On | Cell Line |
| Gene / Protein | Expressed In | Cell Line |
| Gene / Protein | Interacts With | Gene / Protein |
| Cell Line | Carries A | Genotypic Signature |

Each of the triples **7** describes how the subject relates to the object. In the exemplary RDF-based layout, the "Side Effects" of inflammation and vomiting may "Belong To" certain "Disease Classes". In an embodiment, the "Drug" subject may correspond to a specific drug compound **8,** and the "Chemical Structure" object may comprise the simplified molecular-input line-entry system (SMILES) strings or the structural information for that drug compound **8**. A "Drug Combination" subject, corresponding to a specific drug combination **9,** may comprise one or more "Drug" objects. A "Cell Line" may comprise a particular biological unit collected from a patient, such as biological data concerning a patient. A genotypic signature may comprise genetic makeup data for a patient with a particular disease. A "Protein," or the "Gene" used to synthesize that protein, may be "Expressed In" a "Cell Line." The disclosed triples **7** and schemas **3** may provide the advantage of intelligently integrating the knowledge base **1** into structured models and datasets to enable deep learning processes and systems that provide an improved analysis of side effects **10** associated with certain combinations **9** of drug compounds **8**.

The RDF format differs from relational database tables, whose relations are pre-defined at design time and are implicit across the rows and columns of a table. Instead, RDF relationships **5** are explicitly stored as properties. In a graph-based representation, these properties may be associated with the edges **5'** that connect vertices **4'** in the graph **2**. The explicit storage of these relationships **5** provides the context for interpretation of the parameters or attributes **6** for the drug compounds **8**. Further, storage of the relationship 5 in addition to the parameter **6** allows for alteration of the relationship **5** without altering the parameter **6,** and *vice versa*. The independent adjustment of these factors allows the logic to support extensions or changes to the knowledge base **1** via an adjustment of parameters **6** or relationships **5,** rather than using a single degree of freedom. In an embodiment, these storage formats and datasets may benefit the identification and analysis of side effects **10** for drug combinations **9**.

The present disclosure may utilize the enhanced level of structured data offered by knowledge graphs **2** to identify new and useful combinations of information extracted from the existing information received from the knowledge base **1**. To accomplish this result, the present disclosure describes embedding techniques for translating the knowledge graph **2** to a plot of nodes **12'** within an embedding space **11** that represent embedding vectors **12**. These techniques may further include knowledge graph enhancements that comprise selecting an area of interest within the embedding space **11**, identifying empty areas within the area of interest in the embedding space **11**, identifying a center node **12'** from the empty areas, and reconstructing relationships (i.e., edges or connections) of new nodes **12'** that represent the center nodes **12'**. The new node **12'** may correspond to a new triple **7** not represented in the knowledge graph **2**, creating an updated or sequential knowledge graph **2**. Those new nodes **12'** are depictions of the center nodes **12'** from the embedding space **11,** and may represent new drug combinations **9** not expressed in the original knowledge graph **2**.

The features described herein are applicable to knowledge graphs **2** of data representing various fields of medicine, and may represent information within a specific field such as drug combinations **9** and drug side effects **10** for a particular field of diseases **13.** In the example of the knowledge graph enhancement representing drug combination data, the new nodes **4'** in the updated knowledge graph **2** may include a chemical formulation for an existing drug combination **9,** that has been updated with new added drug compounds **8.** However, the knowledge graph **2** is initially generated from information received in the knowledge base **1.** Constructing the updated knowledge graph **2** may include at least two steps. First, a graph schema **3** is obtained for the knowledge graph **2** and a refinement is applied as the knowledge graph **2** is being generated. This defines the types of vertices **4'** and edges **5'** that are generated into the knowledge graph **2.** Second, the knowledge graph **2** is populated with information by ingesting the knowledge base **1** from one or more data sources, and applying one or more knowledge extraction techniques (*e.g.*, natural language processing (NLP), schema mapping, computer visions, or the like) to create the vertices **4'** and edges **5'** in the knowledge graph **2.**

Each data source may create its own data processing pipeline for extracting data to include into the knowledge graph **2** being constructed. The resulting knowledge graph **2** provides a specific format of structured data where each node **4'** includes information, and each connecting edge **5'** represents a relationship 5 between nodes **4'.** For example, **Figure 1** illustrates a knowledge graph schema **3** including information pertaining to known drug compounds **8** and their side effects **10,** where each node **4'** includes information and each edge **5'** represents a relationship **5** between the information included in the nodes **4'.** To provide additional context of the technical field, including network or knowledge graph training, embedding space generation and link prediction, the entire contents of U.S. Patent No. 10,157,226, issued on December 18, 2018, U.S. Patent No. 10,205,734, issued on February 12, 2019, and U.S. Patent No. 10,262,079, issued on April 16, 2019, are hereby incorporated by reference.

In accordance with certain embodiments, an embedding space **11** may be generated from a knowledge graph **2.** Graph embeddings **12** may be used to fully analyze the data for several reasons. Machine learning on graphs is limited in comparison with approaches used in vector spaces **11.** Embedding spaces **11** are compressed representations of the data, which pack node properties in an embedding vector **12,** that are more practical to use in equation operations than an adjacency matrix that describes connections between nodes **4'** in a graph. Further, vector operations are simpler and faster than comparable operations on graphs. Many embedding approaches are known in the art, including factorization approaches, random walk approaches, deep approaches, structural deep network embedding (SDNE) approaches, vertex embedding approaches, and graph embedding approaches. In an embodiment, the approach may comprise: sampling and relabeling sub-graphs around the selected node **4'**; training the model to maximize the probability of predicting a sub-graph that exists in the graph on the input; and computing embedding spaces **11** based on a hidden layer. Accordingly, technical improvements are realized when a computing device structures information into embedding spaces **11** based on knowledge graphs **2** and runs search queries **14** on the embedding spaces **11,** which specifically result in the retrieval of more relevant and accurate information, in a shorter amount of time. Furthermore, calculations may be performed to predict the relationship **5** between a drug side effect **10** and a drug combination **9,** and rank the link predictions **15** using polypharmacy scoring functions **16.**

In some implementations, the embedding vectors **12** may include points **12'** in a k-dimensional embedding space **11,** and may provide latent semantic representations for structured knowledge in the sequential knowledge graph **2.** In some implementations, the embeddings **12** may enable direct explicit relational inferences among entities via simple calculation of embedding vectors **12,** and may be effective at highlighting key concepts underlying sophisticated data. In some implementations, a loss function may be minimized in order to learn optimal parameters that best discriminate positive statements from negative statements. In such implementations, the loss function may include a function that maps a statement **17** onto a real number (*e.g.*, a metric representation **26**) that represents the likelihood of that statement **17** to be true. In such implementations, the loss function may include a pairwise margin-based loss function, a negative log-likelihood loss function, and/or the like. In some implementations, the activity signature platform may assign scores to statements of the sequential knowledge graph **2** in order to aid the loss function in determining how well the sequential knowledge graph **2** tells positive statements from negative statements.

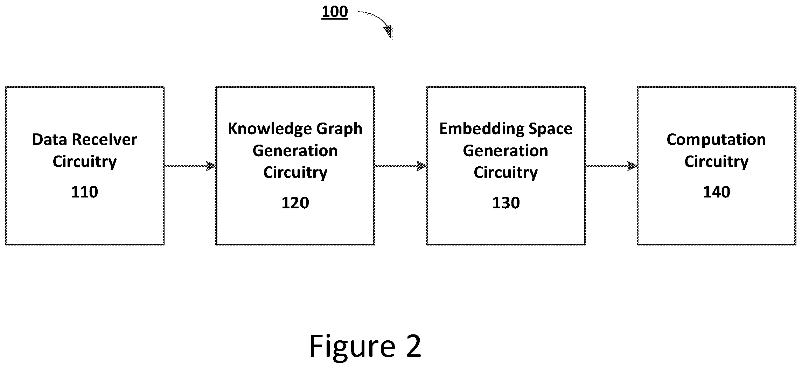

**Figure 2** illustrates an embodiment of a system **100** for implementing polypharmacy scoring. The circuitry described herein may include the hardware, software, middleware, application program interfaces (APIs), and/or other components for implementing the corresponding features of the circuitry. Initially, a data receiver circuitry **110** may be configured to receive drug molecular-structure data **29** and drug side-effect data **30.** A knowledge graph generation circuitry **120** may be configured to generate a graphically represented data structure model based on the received dataset, *e.g.* the knowledge base **1.** The knowledge graph generation circuitry **120** may construct a knowledge graph **2** from the received information **1** that is mapped to a predefined graph schema **3**, in accordance with certain embodiments. The resulting graphical representation provides a specific format of structured data where each connecting edge **5'** between two nodes **4'** represents a relationship **5** between two entities **4.** The knowledge graph generation circuitry **120** may further train the knowledge graph **2,** in accordance with certain embodiments.

The polypharmacy scoring system **100** may further include an embedding space generation circuitry **130** that may be configured to generate embedding spaces **11** based on knowledge graphs **2.** The embedding space generation circuitry **130** may convert the data **4** and relationships **5** represented in the knowledge graph **2** into a plot of nodes **12'** within an embedding space **11**. The generated embedding space **11** may include vector nodes **12'** *(e.g., representations of embedding vectors **12** that may correspond to triples **7**)* representing the structured information included in the knowledge graph **2**.

In some embodiments, the system **100** may include a computation circuitry **140** for implementing computations within the embedding space **11**. For example, the computation circuitry **140** may be configured to: determine a plurality of candidate statements **17**; determine a medical effect weighting **18** from the combination index (CI) **19** based on the drug combination query **14**; determine a polypharmacy score **20** for each candidate statement **17** based on the drug combination query **14** and based on the medical effect weighting **18** using the embedding space **11** of the knowledge graph **2**; and/or, rank the predicted links **15** between the drug combinations **9** and their potential side effects **10**. The computation circuitry **140** may enable the modeling of medical effect weighting **18** for a drug combination **9** in order to score the prediction **15** of the relationship **5** between a drug combination **9** and a drug side effect **10**.

In some embodiments, the candidate statements **17** may represent associations **27** between a plurality of side effects **10** and a plurality of drug combinations **9**. A metric representation **26** may be determined for each candidate statement **17**. Each metric representation **26** may comprise the probability of an association **27** between one of the side effects **10** and one of the drug combinations **9**. The metric representations **26** of the candidate statements **17** may be based on a medical effect weighting **18** of the embeddings **12**. The medical effect weighting **18** may be based on a drug combination query **14**. A polypharmacy score **20** may be determined for each candidate statement **17**. The scores **20** may be based on the metric representations **26**. In certain embodiments, the metric representations **26** of the candidate statements **17** may be ranked, and the polypharmacy scores **20** may be based on the metric representations ranking **28**.

In addition, the computation circuitry **140** may identify gap regions **21** within the region of interest **22**, and compute Max-Min Multi-dimensional computations to determine a center **23** for the gap regions **21** within the region of interest **22**. The computation circuitry **140** is further configured to consider that center **23** to be a center node **12'** in the embedding space **11** that represents a newly discovered drug combination **9** that was not present in the original knowledge graph **2**. This may be technically implemented by generating a new node **12'** within the embedding space **11** at the determined center **23** having the attributes **6** of the newly discovered combination **9**. Overall, executing the polypharmacy scoring process provides improvements to the computing capabilities of a computer device executing the process by reducing the search space and by allowing for more efficient data analysis to analyze large amounts of data in a shorter amount of time.

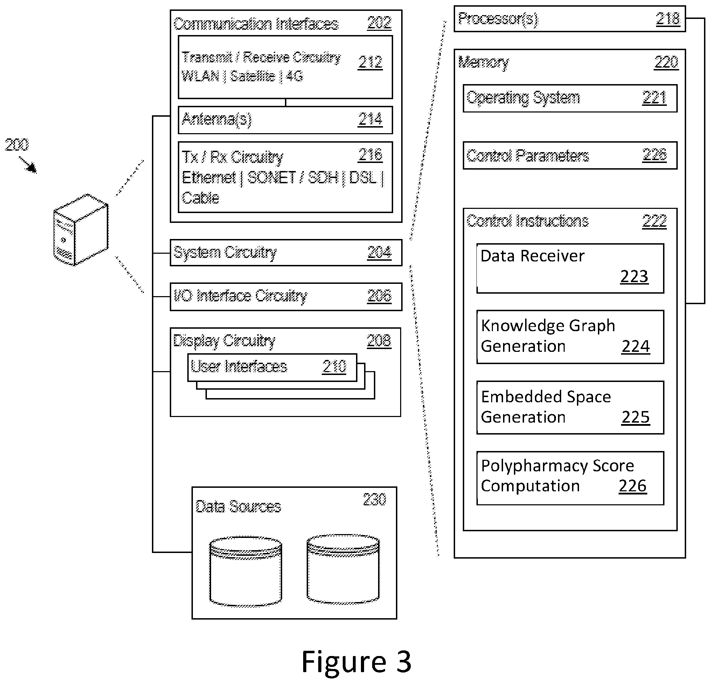

**Figure 3** illustrates a computer architecture of a computer device **200** on which the features of the polypharmacy scoring system **100** may be executed. The computer device **200** includes communication interfaces **202**, system circuitry **204**, input/output (I/O) interface circuitry **206**, and display circuitry **208**. The graphical user interfaces (GUIs) **210** displayed by the display circuitry **208** may be representative of GUIs generated by the system **100** to present a query to an enterprise application or end user, requesting information on a compound **8** to be replace and/or compound attributes **6** desired to be satisfied by a candidate discovery compound **8**. The graphical user interfaces (GUIs) **210** displayed by the display circuitry **208** may also be representative of GUIs generated by the system **100** to receive query inputs identifying the drug compound **8** to be replace and/or compound attributes 6 desired to be satisfied by a candidate discovery compound **8**. The GUIs **210** may be displayed locally using the display circuitry **208**, or for remote visualization, e.g., as HTML, JavaScript, audio, and video output for a web browser running on a local or remote machine. Among other interface features, the GUIs **210** may further render displays of any new formulations resulting from the replacement of compounds(s) **8** with discovery compound(s) **8** selected from the processes described herein.

The GUIs **210** and the I/O interface circuitry **206** may include touch sensitive displays, voice or facial recognition inputs, buttons, switches, speakers and other user interface elements. Additional examples of the I/O interface circuitry **206** includes microphones, video and still image cameras, headset and microphone input / output jacks, Universal Serial Bus (USB) connectors, memory card slots, and other types of inputs. The I/O interface circuitry **206** may further include magnetic or optical media interfaces (*e.g.*, a CDROM or DVD drive), serial and parallel bus interfaces, and keyboard and mouse interfaces.

The communication interfaces **202** may include wireless transmitters and receivers (herein, "transceivers") **212** and any antennas **214** used by the transmit-and-receive circuitry of the transceivers **212**. The transceivers **212** and antennas **214** may support WiFi network communications, for instance, under any version of IEEE 802.11, *e.g.,* 802.11n or 802.11ac, or other wireless protocols such as Bluetooth, Wi-Fi, WLAN, cellular (4G, LTE/A). The communication interfaces **202** may also include serial interfaces, such as universal serial bus (USB), serial ATA, IEEE 1394, lighting port, $I^2C$, slimBus, or other serial interfaces. The communication interfaces **202** may also include wireline transceivers **216** to support wired communication protocols. The wireline transceivers **216** may provide physical layer interfaces for any of a wide range of communication protocols, such as any type of Ethernet, Gigabit Ethernet, optical networking protocols, data over cable service interface specification (DOCSIS), digital subscriber line (DSL), Synchronous Optical Network (SONET), or other protocol.

The system circuitry **204** may include any combination of hardware, software, firmware, APIs, and/or other circuitry. The system circuitry **204** may be implemented, for example, with one or more systems on a chip (SoC), application specific integrated circuits (ASIC), microprocessors, discrete analog and digital circuits, and other circuitry. The system circuitry **204** may implement any desired functionality of the system **100**. As just one example, the system circuitry **204** may include one or more instruction processor **218** and memory **220**.

The memory **220** stores, for example, control instructions **222** for executing the features of the system **100,** as well as an operating system **221**. In one implementation, the processor **218** executes the control instructions **222** and the operating system **221** to carry out any desired functionality for the polypharmacy scoring system **100,** including those attributed to data receiver **223** (*e.g.,* relating to the data receiver circuitry **110**), knowledge graph generation **224** (*e.g.,* relating to the knowledge graph generation circuitry **120**), embedding space generation **225** (*e.g.,* relating to the embedding space generation circuitry **130**), and/or polypharmacy score computation **226** (*e.g.*, relating to the computation circuitry **140**). The control parameters **227** provide and specify configuration and operating options for the control instructions **222,** operating system **221,** and other functionality of the computer device **200**.

The computer device **200** may further include various data sources **230**. Each of the databases that are included in the data sources **230** may be accessed by the system **100** to obtain data for consideration during any one or more of the processes described herein. For example, the data receiver circuitry **110** may access the data sources **230** to obtain the information for generating the knowledge graph **2** and the embedding space **11**. In an embodiment, a data receiver circuitry **110** may be configured to receive a knowledge graph **2**.

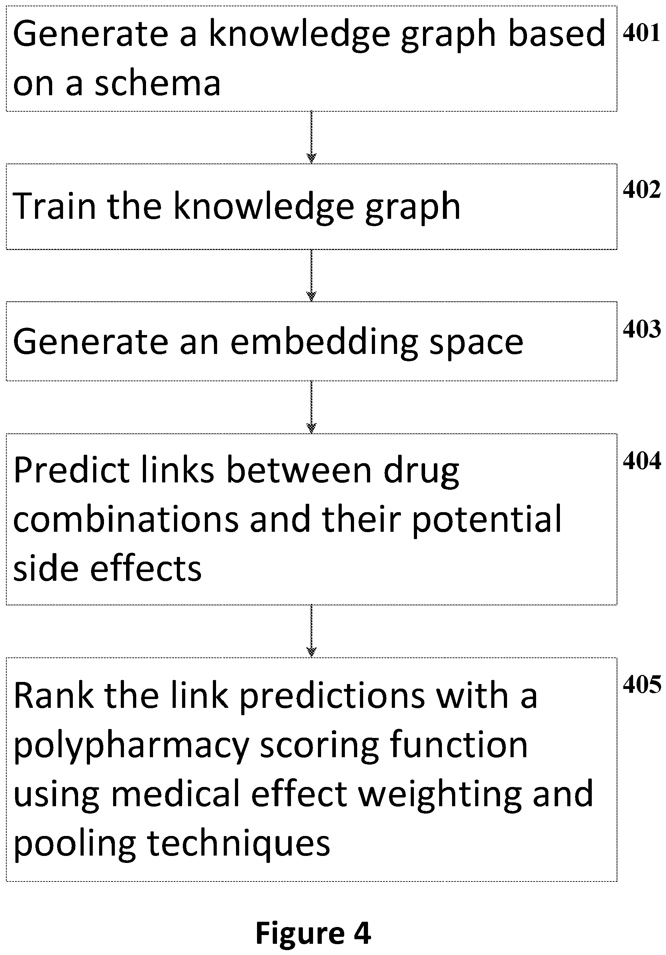

**Figures 4-7** show flowcharts representing processes and methods, as implemented in accordance with certain embodiments. The processes may be implemented by a computing device, system, and/or circuitry components as described herein. **Figure 4** is a flowchart illustrating steps of an embodiment for ranking link predictions via the circuitry described in **Figure 2,** in accordance with certain embodiments of the invention. The prediction rank may be obtained by the following process: generating the knowledge graph **2** based on the schema **3** (block **401**); training the knowledge graph **2** (block **402**); generating an embedding space **11** (block **403**); predicting links or relationships **5** between drug combinations **9** and their potential side effects **10** (block **404**); and, ranking the link predictions **15** with a polypharmacy scoring function **16** using medical effect weighting **18** and pooling techniques **24** (block **404**).

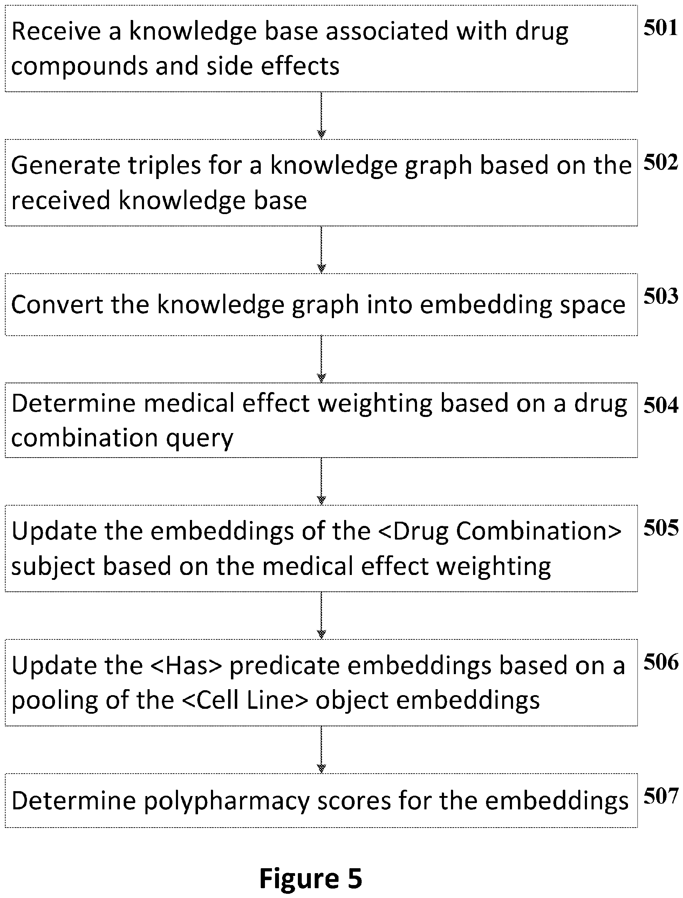

**Figure 5** is a diagram showing flowcharts representing steps in a process or method for determining link predictions and polypharmacy scores via the circuitry described in **Figure 2,** in accordance with certain embodiments of the invention. At block **501,** a knowledge base **1** is received. In embodiment, the knowledge base **1** may be associated with a plurality of drug compounds **8,** drug combinations **9** and their associated side effects **10**. In some embodiments, the received information may comprise an existing knowledge graph **2** with drug molecular-structure data **29** and drug side-effect data **30**. In certain embodiments, the information received from the knowledge base **1** may be received from a combination index **19** of medical side effects for a combination of a plurality of drug compounds **8**. Such a received knowledge base **1** may be training and enhanced by the knowledge graph generation circuitry **120**. The knowledge graph generation circuitry **120** may construct a knowledge graph **2** based on the knowledge base **1**. Triples **7** may be generated for a knowledge graph **2** based on the knowledge base **1** (block **502**). The generated knowledge graph **2** may include nodes **4'** of information, and connecting edges **5'** representing a relationship **5** between nodes **4'** at a head-end of the edge **5'** and at a tail-end of the edge **4"**. Referring back to **Figure 1,** a schema **3** may be utilized by the knowledge graph generation circuitry **120** to generate the knowledge graph **2** with structured data within a scope of known drug compounds **8** and side effects **10**.

The embedding space generation circuitry **130** may receive the knowledge graph **2** and convert it into an embedding space **11,** as represented by block **503**. The embedding space generation circuitry **130** may convert the structured data of the triples **7** from the knowledge graph **2** into embedding vectors **12**. A vector **12** may comprise a metric representation of the triples **7** that have the following format: <head entity **4'**, relationship **5'**, tail entity **4'**> (*e.g.*, <Drug_Combination, has_Category, Side_Effect>). The vector conversion may be applied across the knowledge graph **2**. The embedding space generation circuitry **130** may further implement the embedding space conversion by modeling the triples **7** according to an elaboration of a neural network architecture to learn the representations of the knowledge graph **2**. In this way, the embedding space **11** may be constructed to comprise nodes **12'** (representing the embedding vectors **12**) corresponding to the structured data comprising the knowledge graph **2**. The nodes **12'** depicted in the embedding space **11** may correspond to drug combinations **9** that relate to a side effect **10** included in the knowledge graph **2**.

The computation circuitry **140** may implement a polypharmacy score process. To begin, the computation circuitry **140** may initially receive a drug combination query **14** to identify drug formulations for at least two drug compounds **8** that are desired to be combined and further analyzed. The query **14** may further include desired compound attributes **6** for the new discovery node **5'** that will be determined for inclusion into the formulation. The computation circuitry **140** may determine a medical effect weighting **18** based on the drug combination query **14** (block **504**). Various known methods may be used to determine the medical effect weighting **18**. The method of calculating the medical effect weighting **18** of a drug combination **9** may comprise: a Loewe additivity score method, a Chou-Talalay combination index method, a Tau estimation method, a pharmacological independence method, and/or a Bliss statistical independence method. In an embodiment, the query **14** may be utilized to identify a medical effect weighting **18** using any of the above-mentioned methods.

The computation circuitry **140** may update the effects of a medical effect weighting **18** on the drug combinations **9** (block **505**). In some embodiments, the embedding of the <Drug Combination> subject may be updated based on the medical effect weighting **18.** The <Has> predicate embeddings **12** may be updated based on a pooling method **24** performed on the <Cell Line> object embeddings **12** (block **506**). A polypharmacy score **20** may be determined for the new embeddings **12** that were updated based on the drug combination query **14,** the medical effect weighting **18** and the pooling **24** (block **507**).

In an embodiment, the polypharmacy scoring function **16** may utilize updated embeddings **12** of drug combination **9** based on a multiplication of a Loewe additivity score **18**. The pooling **24** between the <Has> predicate and the <Cell Line> object may comprise element-wise multiplication the embeddings **12** of a side effect **10** and the embeddings **12** of the cell lines **25**. From the calculated polypharmacy scores **20** for new links, the node **12'** with the highest discovery score **20** may be selected as the discovery node **12',** and the corresponding drug combination **9** may be selected for further experimental testing.

In an embodiment, the polypharmacy scoring function **16** may comprise an improvement of complex scoring function. The function may be based on the predicate embeddings **12** for a <Drug Combination> subject (*e.g.*, A+B may represent the combination of drug A and drug B), embeddings **12** of a <Cell Line> subject (*e.g.,* C1 may represent a cell line **25** for testing the drug combination **9**) and embeddings **12** of an <Side Effect> object (*e.g.,* EI may represent a side effect **10** of the drug combination **9**). In some embodiments, the medical effect weighting **18** of a drug combination **9** may be calculated based on the concentrations for two drug compounds **8** (*e.g.,* a and b may represent the drug concentrations for drug A and drug B, respectively) and the effective dosages for the two compounds **8** (*e.g.,* ED(A) and ED(B) may represent the effective dosages for drug A and drug B, respectively). In an embodiment, the medical effect weighting **18** may be calculated using the Loewe additivity score equation

$$fLoewe = l(A+B) = \frac{a}{ED(A)} + \frac{b}{ED(B)}$$

which may be multiplied with the drug combination **9,**

$$l(A+B)(A+B))$$

in order to update the embeddings **12** of the drug combination **9**. The predicate embeddings **12** for the cell lines **25** may be updated by pooling **24** between the linked relationships **5** and the cell line **25**. Such pooling **24** may comprise element-wise multiplication the embeddings **12** of a side effect **10** and the embeddings **12** of the cell lines **25**. A polypharmacy score **20** for new prediction links **15** may be calculated using the following equations:

$$fPolyPharm = Re(< l(A+B)(A+B), P(has, cell\_line\ C1), side\_effect\ E1 >)$$

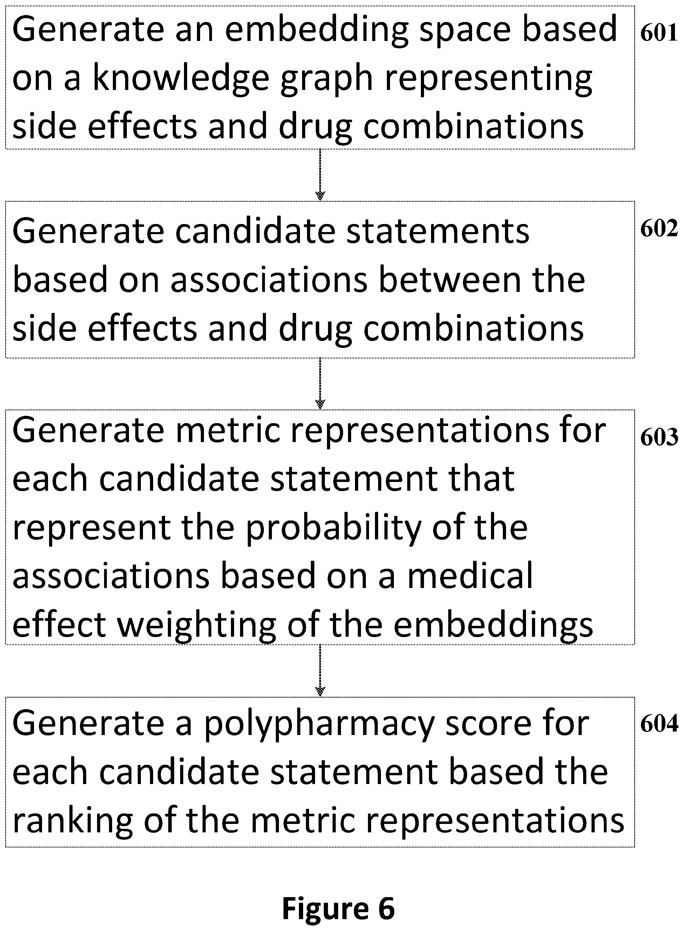

As shown in **Figure 6,** an embodiment for the disclosed products, systems and methods may include the generation (block **601**) of embeddings **12** based on knowledge graphs **2** that represent a knowledge base **1** of drug combinations **9** and side effects **10**. The disclosed systems and methods may include the generation (block **602**) of candidate statements **17** that may represent associations **27** between the side effects **10** and the drug combinations **9,** and the generation (block **603**) of metric representations **26** for each candidate statement **17**. Each metric representation **26** may comprise the probability of an association **27** of a particular side effect **10** with a drug combination **9**. The metric representations **26** may be generated using a medical effect weighting **18** of the embeddings **12**. The medical effect weighting **18** may be based on a drug combination query **14**. In an embodiment, the disclosed systems and methods may include the generation (block **604**) of a polypharmacy score **20** for each candidate statement **17**. The scores **20** may be based on the ranking **28** of the metric representations **26**.

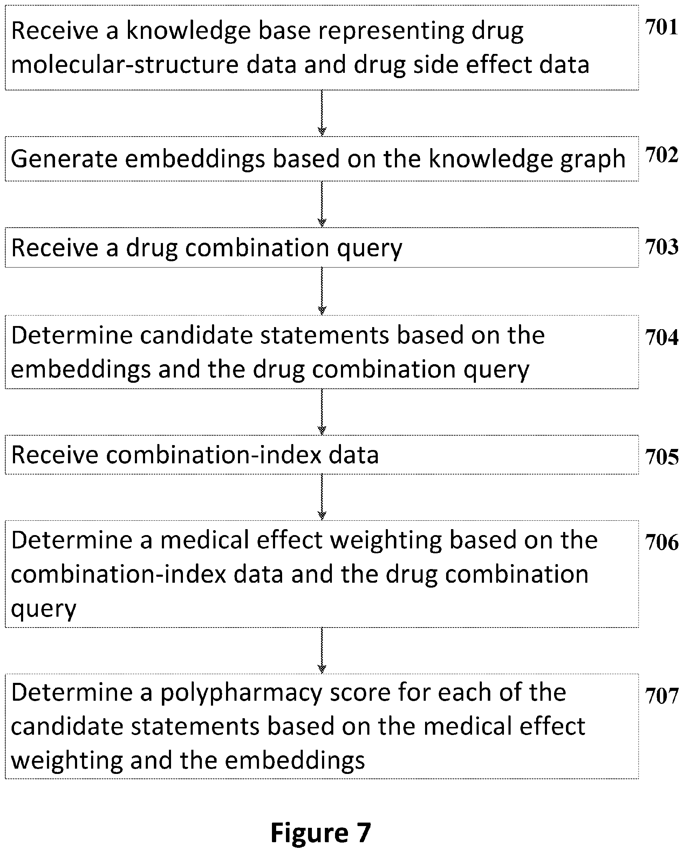

**Figure 7** illustrates another embodiment for the disclosed products, systems and methods. A knowledge graph **2** may be received (block **701**) via a knowledge graph receiving circuitry **110**. The knowledge graph **2** may represent drug molecular-structure data **29** and drug side-effect data **30**. In certain embodiments, this data **29** and **30** may correspond to a plurality of drug combinations **9**. The knowledge graph **2** may be based on a knowledge base **1**. In some embodiments, the knowledge base **1** may comprise training data represented by an existing knowledge graph **2**. The existing knowledge graph **2** may represent drug molecular-structure data **29** and drug side-effect data **30**.

In an embodiment, the computation circuitry **140** may be configured to train the existing knowledge graph **2** and generate the knowledge graph **2** received by the knowledge graph receiving circuitry **110**. In some embodiments, the knowledge graph **2** may be generated using a schema **3** providing node representations **4"** for drug compound data, chemical substructure data, drug combination data, gene data, cell line data, genotypic data, side-effect data, and disease classification data. The knowledge base **1** may comprise drug compound data, chemical substructure data, drug combination data, gene data, cell line data, genotypic data, side-effect data, and disease classification data.

At block **702**, embeddings **12** may be generated based on the knowledge graph **2**. The embeddings **12** may be generated via an embedding-space generation circuitry **130**. A drug combination query **14** may be received (block **703**) by a computation circuitry **140**. A plurality of candidate statements **17** may be determined (block **704**) based on the embeddings **12** and the drug combination query **14**. The candidate statements **17** may represent associations **27** between a plurality of side effects **10** and a plurality of drug combinations **9**. The candidate statements **17** may be determined via the computation circuitry **140**.

In certain embodiments, combination-index data **31** may be received (block **705**). The combination-index data **31** may represent medical effects of a combination **9** of drug compounds **8**. The combination-index data **31** may be received by the computation circuitry **140**. At block **706**, a medical effect weighting **18** may be determined based on the combination-index data **31** and the drug combination query **14**. The medical effect weighting **18** may be determined by the computation circuitry **140**. The medical effect weighting **18** may be determined based on one of the following methods: a Loewe additivity score method, a Chou-Talalay combination index method, a Tau estimation method, a pharmacological independence method, and a Bliss statistical independence method.

A polypharmacy score **20** may be determined (block **707**) for each of the candidate statements **17** based on the medical effect weighting **18** and the embeddings **12**. Each polypharmacy score **20** may be a metric representation **26** comprising a probability of an association **27** between one of the side effects **10** and one of the drug combinations **9**. The polypharmacy scores **20** may be determined via the computation circuitry **140**. In some embodiments, the computation circuitry **140** may be configured to rank the metric representations **26** of the candidate statements **17**. The computation circuitry **140** may be further configured to adjust the embeddings **12** based on a pooling **24** of the embeddings **12**.

While the present disclosure has been particularly shown and described with reference to an embodiment thereof, it will be understood by those skilled in the art that various changes in form and details may be made therein without departing from the spirit and scope of the present disclosure. Although some of the drawings illustrate a number of operations in a particular order, operations that are not order-dependent may be reordered and other operations may be combined or broken out. While some reordering or other groupings are specifically mentioned, others will be apparent to those of ordinary skill in the art and so do not present an exhaustive list of alternatives.

## Claims

1. A system for identifying side effects for drug combinations, comprising:

   a knowledge graph receiver circuitry configured to receive a knowledge graph based on a knowledge base, the knowledge graph representing drug molecular-structure data and drug side-effect data;
   an embedding-space generation circuitry configured to convert the knowledge graph into embeddings; and,
   a computation circuitry configured to receive a drug combination query, the computation circuitry configured to determine a plurality of candidate statements based on the embeddings and the drug combination query, the candidate statements representing associations between a plurality of side effects and a plurality of drug combinations, the computation circuitry configured to receive combination-index data, the combination-index data representing medical effects of a combination of drugs, the computation circuitry configured to determine a medical effect weighting based on the combination-index data and the drug combination query, the computation circuitry configured to determine a polypharmacy score for each candidate statement based on the medical effect weighting and the embeddings, each polypharmacy score being a metric representation comprising a probability of an association between one of the side effects and one of the drug combinations.

2. The system of claim 1, wherein the knowledge base is an existing knowledge graph representing drug molecular-structure data and drug side-effect data; and/or
   wherein the knowledge base comprises drug compound data, chemical substructure data, drug combination data, gene data, cell line data, genotypic data, side-effect data, and disease classification data.

3. The system of claim 1 or claim 2, wherein the knowledge graph is generated using a schema providing node representations for drug compound data, chemical substructure data, drug combination data, gene data, cell line data, genotypic data, side-effect data, and disease classification data.

4. The system of any preceding claim, wherein the computation circuitry is further configured to rank the metric representations of the candidate statements; and/or
   wherein the computation circuitry is further configured to adjust the embeddings based on a pooling of the embeddings.

5. The system of any preceding claim, wherein the medical effect weighting is based on a method selected from a group consisting of: a Loewe additivity score method, a Chou-Talalay combination index method, a Tau estimation method, a pharmacological independence method, and a Bliss statistical independence method.

6. A method for identifying side effects for drug combinations, comprising:

   receiving a knowledge graph representing drug molecular-structure data and drug side-effect data, wherein the knowledge graph is based on a knowledge base, wherein the knowledge graph is received via a knowledge graph receiving circuitry;
   generating embeddings based on the knowledge graph, wherein the embeddings are generated via an embedding-space generation circuitry;
   receiving a drug combination query, wherein the drug combination query is received via a computation circuitry;
   determining a plurality of candidate statements based on the embeddings and the drug combination query, wherein the candidate statements represent associations between a plurality of side effects and a plurality of drug combinations, wherein the candidate statements are determined via the computation circuitry;
   receiving combination-index data, wherein the combination-index data represents medical effects of a combination of drugs, wherein the combination-index data is received via the computation circuitry;
   determining a medical effect weighting based on the combination-index data and the drug combination query, wherein the medical effect weighting is determined via the computation circuitry; and,
   determining a polypharmacy score for each of the candidate statements based on the medical effect weighting and the embeddings, wherein each polypharmacy score is a metric representation comprising a probability of an association between one of the side effects and one of the drug combinations, wherein the polypharmacy scores are determined via the computation circuitry.

7. The method of claim 6, wherein the knowledge base is an existing knowledge graph representing drug molecular-structure data and drug side-effect data; and/or
   wherein the knowledge base comprises drug compound data, chemical substructure data, drug combination data, gene data, cell line data, genotypic data, side-effect data, and disease classification data.

8. The method of claim 6 or claim 7, wherein the knowledge graph is generated using a schema providing node representations for drug compound data, chemical substructure data, drug combination data, gene data, cell line data, genotypic data, side-effect data, and disease classification data.

9. The method of any one of claims 6 - 8, wherein the computation circuitry is further configured to rank the metric representations of the candidate statements; and/or
   wherein the computation circuitry is further configured to adjust the embeddings based on a pooling of the embeddings.

10. The method of any one of claims 6 - 9, wherein the medical effect weighting is based on a method selected from a group consisting of: a Loewe additivity score method, a Chou-Talalay combination index method, a Tau estimation method, a pharmacological independence method, and a Bliss statistical independence method.

11. A product for identifying side effects for drug combinations, comprising:

   a machine-readable medium, other than a transitory signal; and,
   instructions stored on the machine-readable medium, the instructions configured to, when executed, cause processing circuitry to:

   receive a knowledge graph representing drug molecular-structure data and drug side-effect data, wherein the knowledge graph is based on a knowledge base, wherein the knowledge graph is received via a knowledge graph receiving circuitry;

generate embeddings based on the knowledge graph, wherein the embeddings are generated via an embedding-space generation circuitry;

receive a drug combination query, wherein the drug combination query is received via a computation circuitry;

determine a plurality of candidate statements based on the embeddings and the drug combination query, wherein the candidate statements represent associations between a plurality of side effects and a plurality of drug combinations, wherein the candidate statements are determined via the computation circuitry;

receive combination-index data, wherein the combination-index data represents medical effects of a combination of drugs, wherein the combination-index data is received via the computation circuitry;

determine a medical effect weighting based on the combination-index data and the drug combination query, wherein the medical effect weighting is determined via the computation circuitry; and,

determine a polypharmacy score for each of the candidate statements based on the medical effect weighting and the embeddings, wherein each polypharmacy score is a metric representation comprising a probability of an association between one of the side effects and one of the drug combinations, wherein the polypharmacy scores are determined via the computation circuitry.

12. The product of claim 11, wherein the knowledge base is an existing knowledge graph representing drug molecular-structure data and drug side-effect data; and/or

wherein the knowledge base comprises drug compound data, chemical substructure data, drug combination data, gene data, cell line data, genotypic data, side-effect data, and disease classification data.

13. The product of claim 11 or claim 12, wherein the knowledge graph is generated using a schema providing node representations for drug compound data, chemical substructure data, drug combination data, gene data, cell line data, genotypic data, side-effect data, and disease classification data.

14. The product of any one of claims 11- 13, wherein the computation circuitry is further configured to rank the metric representations of the candidate statements; and/or

wherein the computation circuitry is further configured to adjust the embeddings based on a pooling of the embeddings.

15. The product of any one of claims 11- 14, wherein the medical effect weighting is based on a method selected from a group consisting of: a Loewe additivity score method, a Chou-Talalay combination index method, a Tau estimation method, a pharmacological independence method, and a Bliss statistical independence method.

Figure 1

EP 3 731 239 A1

100

| Data Receiver Circuitry 110 | → | Knowledge Graph Generation Circuitry 120 | → | Embedding Space Generation Circuitry 130 | → | Computation Circuitry 140 |

Figure 2

Figure 3

EP 3 731 239 A1

Generate a knowledge graph based on a schema | 401

Train the knowledge graph | 402

Generate an embedding space | 403

Predict links between drug combinations and their potential side effects | 404

Rank the link predictions with a polypharmacy scoring function using medical effect weighting and pooling techniques | 405

**Figure 4**

Receive a knowledge base associated with drug compounds and side effects | 501

Generate triples for a knowledge graph based on the received knowledge base | 502

Convert the knowledge graph into embedding space | 503

Determine medical effect weighting based on a drug combination query | 504

Update the embeddings of the <Drug Combination> subject based on the medical effect weighting | 505

Update the <Has> predicate embeddings based on a pooling of the <Cell Line> object embeddings | 506

Determine polypharmacy scores for the embeddings | 507

**Figure 5**

Generate an embedding space based on a knowledge graph representing side effects and drug combinations | 601

Generate candidate statements based on associations between the side effects and drug combinations | 602

Generate metric representations for each candidate statement that represent the probability of the associations based on a medical effect weighting of the embeddings | 603

Generate a polypharmacy score for each candidate statement based the ranking of the metric representations | 604

**Figure 6**

Receive a knowledge base representing drug molecular-structure data and drug side effect data | 701

Generate embeddings based on the knowledge graph | 702

Receive a drug combination query | 703

Determine candidate statements based on the embeddings and the drug combination query | 704

Receive combination-index data | 705

Determine a medical effect weighting based on the combination-index data and the drug combination query | 706

Determine a polypharmacy score for each of the candidate statements based on the medical effect weighting and the embeddings | 707

**Figure 7**

Europäisches
Patentamt

European
Patent Office

Office européen
des brevets

# EUROPEAN SEARCH REPORT

Application Number

EP 20 16 4511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARINKA ZITNIK ET AL: "Modeling polypharmacy side effects with graph convolutional networks", ARXIV.ORG, CORNELL UNIVERSITY LIBRARY, 201 OLIN LIBRARY CORNELL UNIVERSITY ITHACA, NY 14853, 2 February 2018 (2018-02-02), XP081227514, DOI: 10.1093/BIOINFORMATICS/BTY294 | 1-4,6-9, 11-14 | INV. G16H70/40 |
| Y | * Abstract; Chapter 4.1, Chapter 8; figures 1, 3 * | 5,10,15 | |
| X | WILLIAM L HAMILTON ET AL: "Embedding Logical Queries on Knowledge Graphs", ARXIV.ORG, CORNELL UNIVERSITY LIBRARY, 201 OLIN LIBRARY CORNELL UNIVERSITY ITHACA, NY 14853, 5 June 2018 (2018-06-05), XP081197176, * page 3, Example 1; page 13, Appendix B; figure 2a * | 1,6,11 | |
| Y | BOUCHER A N ET AL: "Mathematical formulation of additivity for antimicrobial agents", DIAGNOSTIC MICROBIOLOGY AND INFECTIOUS DISEASE, ELSEVIER, AMSTERDAM, NL, vol. 55, no. 4, 1 August 2006 (2006-08-01) , pages 319-325, XP025146288, ISSN: 0732-8893, DOI: 10.1016/J.DIAGMICROBIO.2006.01.024 [retrieved on 2006-08-01] * abstract * | 5,10,15 | TECHNICAL FIELDS SEARCHED (IPC) G16H |

The present search report has been drawn up for all claims

| Place of search | Date of completion of the search | Examiner |
|---|---|---|
| Munich | 5 August 2020 | Schmidt, Karsten |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62838074
- US 10157226 B
- US 10205734 B
- US 10262079 B